# EUROPÄISCHE PATENTSCHRIFT

(11) **EP 1 234 598 B1**
(45) Veröffentlichungstag und Bekanntmachung des Hinweises auf die Patenterteilung: **28.09.2005**
(21) Anmeldenummer: 02001638.2
(22) Anmeldetag: 24.01.2002
(51) Int. Cl.: A61N 2/02, A61N 1/40

(54) **Magnetspulenanordnung eines Magnetfeldapplikators zur Aufheizung von magnetischen oder magnetisierbaren Substanzen oder Festkörpern in biologischem Gewebe**
Magnetic coil device used in a magnetic field applicator for heating magnetic or magnetisable substances or solid state bodies in a biological tissue
Bobine magnétique utilisée sur un applicateur de champ magnétique pour chauffer des substances ou corps solides magnétiques ou magnétisables disposés dans un tissu biologique

(30) Priorität: 24.02.2001 DE 10109105
(43) Veröffentlichungstag der Anmeldung: 28.08.2002
(73) Patentinhaber: MFH Hyperthermiesysteme GmbH, 14050 Berlin (DE)
(72) Erfinder: Feucht, Peter, 10827 Berlin (DE)
(74) Vertreter: Neubauer, Hans-Jürgen

(56) Entgegenhaltungen:
- WO-A-01/10501
- DE-A- 3 422 930
- FR-A- 1 043 701
- US-A- 4 378 548

## Beschreibung

Die Erfindung betrifft eine Magnetspulenanordnung eines Magnetfeldapplikators zur Aufheizung von magnetischen oder magnetisierbaren Substanzen oder Festkörpern in biologischem Gewebe nach dem Oberbegriff des Anspruchs 1.

Krebserkrankungen können in allgemein bekannter Weise mittels Hyperthermieverfahren behandelt werden, wobei Krebsgewebe gezielt auf Temperaturen von ca. 41°C-46°C für eine irreversible Schädigung aufgeheizt wird.

Bei einem bekannten Hyperthermieverfahren (WO 97/43005) zur Tumortherapie werden magnetisierbare Mikrokapseln verwendet, die durch den Blutstrom in den Tumorbereich gelangen. Diese Mikrokapseln werden bei einer Behandlung mit einem außerhalb eines Patienten erzeugten Magnetwechselfeld beaufschlagt, wobei durch Hystereseeffekte Wärme für eine Hyperthermie in den Mikrokapseln entsteht. Zur Befeldung wird ein lineares Magnetwechselfeld verwendet mit einer Frequenz im Bereich zwischen 10 kHz bis 500 kHz. Die Mikrokapseln sollen ein hochmagnetisierbares Material enthalten, so dass die für die Befeldung erforderliche Stärke des Magnetwechselfeldes, der erforderliche apparative Aufbau, das erforderliche Kühlsystem sowie die elektrische Energieversorgung beherrschbar werden. Ein konkreter apparativer Aufbau ist jedoch nicht angegeben.

In einem weitgehend ähnlichen bekannten Hyperthermieverfahren (EP 0 913 167 A2) werden zur Befeldung rotierende Magnetfelder mit einer Frequenz im Bereich größer 10 kHz verwendet. Zur Erzeugung des hier verwendeten rotierenden Magnetwechselfeldes ist ein Magnetfeldapplikator lediglich skizzenhaft und schematisch angegeben.

Eine gattungsgemäße Magnetspulenanordnung ist in der (nachveröffentlichten) Schrift DE 199 37 492 gezeigt. Der dargestellte Magnetfeldapplikator zur Aufheizung von magnetischen oder magnetisierbaren Substanzen oder Festkörpern in biologischem Gewebe weist ein kühlbares Magnetjoch auf mit zwei gegenüberliegenden, durch einen Befeldungsspalt beabstandeten Polschuhen am Magnetjoch. Zur Erzeugung eines Magnetwechselfeldes sind zwei, jeweils einem Polschuh zugeordnete Magnetspulen vorgesehen, die als Scheibenspulen mit schneckenförmig verlaufenden Spulenwindungen ausgebildet sind und jeweils das Polschuhende der zugeordneten Polschuhe mit einem dazwischenliegenden umlaufenden Magnetspule/Polschuh-Spalt ringförmig umfassen. Konkret bestehen das Magnetjoch und die Polschuhe aus zusammengesetzten und zusammenmontierten Ferritbausteinen.

Für die Hyperthermie, insbesondere mit magnetischen Flüssigkeiten, werden Wechselfeldstärken von ca. 15 bis 20 kA/m bei ca. 50 bis 100 kHz benötigt. Bei einem Befeldungsvolumen von 8 bis 30 l sind dabei von einer Hyperthermieanlage Wirkleistungen von ca. 18 kW bis 80 kW aufzubringen. Diese Energie muss als Hochfrequenz erzeugt werden und dann als Wärme durch Kühlung wieder abgeführt werden, da für die Hyperthermie im Körper eines Patienten nur einige Watt in der magnetischen Flüssigkeit entstehen. Für die Kühlung der Ferritbausteine des Magnetjochs und der Polschuhe sind Maßnahmen mit einer Kühlluftströmung in Kühlspalten angegeben. Die Art der Kühlung der Magnetspulen ebenso wie deren Halterung ist dagegen offen gelassen. Die Kühlung der Magnetspulen ist jedoch problematisch, da hier eine besonders hohe Verlustleistung vorliegt, die je Volumeneinheit höher ist als die Verlustleistung in den Ferritbausteinen und da für Kühlvorrichtungen und Halterungen ein vorgegebener, nur relativ kleiner Einbauraum im Magnetspulenbereich zur Verfügung steht.

Aufgabe der Erfindung ist es daher eine gattungsgemäße Magnetspulenanordnung eines Magnetfeldapplikators zur Aufheizung von magnetischen oder magnetisierbaren Substanzen oder Festkörpern in biologischem Gewebe so weiterzubilden, dass eine effektive Kühlung der Magnetspulen in Verbindung mit einer kompakten Anordnung und Halterung möglich ist.

Diese Aufgabe wird mit den Merkmalen des Anspruchs 1 gelöst.

Gemäß Anspruch 1 ist jeweils die Magnetspule in einem den zugeordneten Polschuh ringförmig umfassenden Spulenkasten angebracht. Der Spulenkasten weist wenigstens eine Kühllufteinströmöffnung zum Anschluss einer Kühlluftpumpe und wenigstens eine Kühlluft-Ausströmöffnung auf.

Damit können vorteilhaft die Magnetjochkühlung und die Magnetspulenkühlung entkoppelt und den unterschiedlichen Kühlanforderungen hinsichtlich der Kühlluftmenge, des Kühlluftdrucks, des Kühlluftdurchsatzes und der Kühlluftströmung optimal angepasst werden. Zudem kann der Spulenkasten zusätzlich zu seiner Aufgabe als Teil der Magnetspulen-/Kühlvorrichtung in einer weiteren Funktion für die mechanische Halterung der Magnetspule verwendet werden. Somit ergibt sich eine vorteilhaft kompakte Bauweise, die für die beengten Platzverhältnisse an einem Magnetfeldapplikator im Bereich des Befeldungsspaltes und des Patientenraumes gut geeignet ist.

Bei einer besonders bevorzugten konkreten Ausführungsform nach Anspruch 2 bestehen das Magnetjoch und die Polschuhe aus zusammenmontierten Ferritbausteinen. Das Magnetjoch ist dabei aus quaderförmigen Ferritbausteinen zusammengesetzt deren Flächen ggf. von Sinterschichten befreit und plan-parallel geschliffen sind. Die quaderförmigen Ferritbausteine bestehen aus aneinandergereihten, im Magnetjoch längs des Magnetflusses ausgerichteten Ferritplatten, die quer zum Magnetfluss durch Isolations-/Kühlungs-Spalte voneinander getrennt sind, durch die Kühlluft für die Magnetjochkühlung leitbar ist. Längs des Magnetflusses liegen die Ferritplatten über nur schmale Anlagespalte aneinander. Zur Bildung der Isolations-/Kühlungs-Spalte sind zwischen die Ferritplatten Separatoren aus Kunststoff eingefügt wobei durch Verkleben der Ferritplatten und der Separatoren die quaderförmigen Ferritbausteine gebildet sind.

Die Polschuhe sind in einer Draufsicht kreisrund ausgeführt und bestehen in einem ähnlichen Aufbau aus keilförmigen Ferritbausteinen, die wie Tortenstücke zusammengesetzt sind. Auch zwischen diesen Ferritbausteinen sind mittel Separatoren lsolations-/Kühlungs-Spalten für eine Polschuhkühlung vorgesehen.

Die in den Ferritbausteinen beim Betrieb eines Magnetfeldapplikators entstehenden Verlustleistungen sind so hoch, dass sie durch Einleitung von Kühlluft in geeignet angelegte Isolations-/Kühlungs-Spalten zwischen den Ferritbausteinen abgeführt werden. Es hat sich jedoch gezeigt, dass eine grundsätzlich mögliche Einbindung der Magnetspulenkühlung in die Vorrichtung für die Magnetjoch- und Polschuhkühlung konstruktiv schwierig, aufwendig und uneffektiv ist. Ein Problem bei einer an sich möglichen Einbindung der Magnetspulenkühlung in die Magnetjoch- und Polschuhkühlung besteht darin, dass die Magnetspule im Vergleich je Volumeneinheit eine höhere Verlustleistung produziert. Mit der erfindungsgemäßen Anordnung und Entkopplung der Kühlsysteme ergeben sich somit insbesondere bei der konkreten Ausführungsform nach Anspruch 2 erhebliche Vorteile für die Anordnung, die Dimensionierung und den Betrieb der beiden Kühlsysteme. Durch den einfachen Aufbau sind zudem der Aufwand für Montagen, Handhabungen und Wartungen sowie die Betriebskosten reduziert.

Gemäß Anspruch 3 sind die Polschuhendflächen jeweils durch eine Polschuhplatte abgedeckt. Die Polschuhplatte überragt dabei seitlich umlaufend die zugeordnete Polschuhendfläche und bildet dabei eine befeldungsspaltseitige Spulenkasten-Bodenwand. Zwischen der Polschuhendfläche und der Polschuhplatte sind zur Bildung von lsolations-/Kühlungs-Spalten Separatoren eingefügt. Diese Separatoren sind im Vergleich zur Anlagefläche der bevorzugt keilförmigen Ferritbausteine relativ klein, so dass ein radial zwischen der Polschuhendfläche und der Polschuhplatte geführter Kühlluftstrom durch die Separatoren nur wenig behindert ist.

In einer konstruktiven Weiterführung nach Anspruch 4 weist die Polschuhplatte im Bereich der Polschuhendfläche eine Vertiefung mit geringerer Materialstärke als der angrenzende Bereich der Spulenkasten-Bodenwand auf. In diese Vertiefung taucht die Polschuhendfläche etwas ein, wobei die umlaufende Kante der Polschuhendfläche abgerundet ist und zwischen der Polschuhplatte und der Polschuhendfläche ein umlaufender Ringspalt als Kühlluftaustritt hergestellt ist. In diesem Ringspalt ist eine Umleitung der radialen Kühlluftströmung zu einer axialen Austrittrichtung möglich. Die Polschuhplatte ist aus Isoliermaterial herzustellen. Grundsätzlich kann dazu Glas verwendet werden. Vorzugsweise wird jedoch hochwertiger glasfaserverstärkter Kunststoff eingesetzt und die vorstehend genannte Vertiefung kann durch Ausfräsen hergestellt werden.

In einer konstruktiv einfach herstellbaren und funktionellen Ausführungsform nach Anspruch 5 sind die Polschuhe in einer Draufsicht kreisrund und entsprechend die Magnetspulen kreisringförmig ausgeführt. Die zugeordneten Spulenkästen sollen jedoch bezüglich der Außenmaße quaderförmig ausgebildet sein und die Polschuhenden sowie die darüber gesteckten Magnetspulen umfassen. Eine quaderförmige Ausbildung der Spulenkästen führt einerseits zu einer einfachen Herstellung, da keine gebogenen Wandteile miteinander verbunden werden müssen. Zudem ergibt sich dabei eine günstige Anordnung von Kühlluft-Einströmöffnungen, die sowohl an den Spulenkasten-Seitenwänden und/oder in vorzugsweise zwei gegenüberliegenden Eckbereichen der Spulenkasten-Deckelwand angeordnet sein können. Dort können bei kühltechnisch günstigen Einströmverhältnissen mit geringem Aufwand die erforderlichen Öffnungen sowie Flanschanschlüsse für anzuschließende Kühlschläuche geschaffen werden.

In einer besonders bevorzugten Weiterbildung nach Anspruch 6 weist die Magnetspule eine Stützstruktur für die Windungen auf. Im Bereich der Magnetspule sind dazu stegförmige, bodenseitige Spulenträger und darüber liegend stegförmige, deckelseitige Spulenträger als einander zugeordnete Spulenträgerpaare vorgesehen, die radial strahlenförmig und gegeneinander winkelversetzt angeordnet sind. Die zugeordneten Spulenträgerpaare sind jeweils durch radial beabstandete und etwa axial ausgerichtete Isolierstäbe verbunden dergestalt, dass zwischen den Isolierstäben Aufnahmefächer gebildet sind in denen die schneckenförmig verlaufenden Spulenwindungen aufgenommen und gehalten sind. Durch die Steghöhe der bodenseitigen Spulenträger sind die Spulenwindungen unter Bildung eines radial äußeren Kühlluft-Eintrittringspalts gegenüber der Spulenkasten-Bodenwand abgehoben. Kühlluft kann dann durch diesen Kühlluft-Eintrittringspalt und durch die mittels der Isolierstäbe gebildeten Zwischenspalte zwischen den Spulenwindungen weiter axial geführt werden. Als Kühlluft-Ausströmöffnung wird ein Deckelausschnitt, vorzugsweise ein kreisförmiger Deckelausschnitt zwischen den deckelseitigen und nicht abgedeckten Spulenträgern über den Spulenwindungen verwendet.

Die Höhe und Länge der Spulenträger sowie der Isolierstäbe sind so zu wählen, dass einerseits die Windungen ausreichend gestützt und gehalten sind und andererseits die Isolationsabstände zwischen den Windungen den Vorschriften über Luft- und Kriechstrecken genügen sowie ausreichend Kühlluft dazwischen durchführbar ist. Besonders günstige Verhältnisse ergeben sich nach Anspruch 7 wenn die Isolierstäbe als Keramikrundstäbe ausgebildet sind. Eine praktisch mit guten Ergebnissen getestete Stützstruktur besteht aus 16 Spulenträgerpaaren mit jeweils sechs Isolierstäben wodurch sich jeweils fünf Windungsaufnahmefächer ergeben. Die Spulenwindungen sind aus einem Band hochfeiner HF-Litzen ausgeführt. Der Spulenkasten und die Stützstruktur für die Magnetspule können für den oberen und den unteren Polschuh jeweils gleich hergestellt sein. Da jedoch bei der Anordnung am unteren Polschuh die Polschuhplatte nach oben weist tragen hier die jeweils deckelseitigen Spulenträger das Gewicht der zugeordneten Magnetspule.

In einer konstruktiv vorteilhaften Weiterbildung nach Anspruch 8 sind aus der Spulenkasten-Bodenwand, den Spulenkasten-Seitenwänden, der Spulenkasten-Deckelwand sowie einer dünnwandigen Windkasten-Innenwand ein umlaufender Windkasten mit einem bodenseitigen Kühlluft-Eintritt-Ringspalt ausgebildet. Zudem ist die Magnetspule radial innen von einer Luftführungswand umgeben wodurch zwischen dieser Luftführungswand und einer benachbarten Polschuhwand ein Ringspalt zum Austritt der Polschuh-Kühlluft hergestellt ist. Dabei sind die Polschuh-Kühlluft und die Magnetspulen-Kühlluft im Bereich dieser Luftführungswand voneinander getrennt und vorteilhaft entkoppelt. Der Windkasten kann als Druckkammer mit Kühlluft beaufschlagt werden, die dann über den bodenseitigen Kühlluft-Eintritt-Ringspalt den unteren Spulenwindungsbereichen, wo die größte Erwärmung der Magnetspule entsteht, bevorzugt zugeführt wird und anschließend zwischen den Spulenwindungen abströmt.

Eine weitere wesentliche Verbesserung der Magnetspulenkühlung ergibt sich mit den Merkmalen des Anspruchs 9. Gemäß Anspruch 9 sind die Spulenträger keilförmig dergestalt ausgebildet, dass die radial äußeren Spulenwindungen mit ihren bodenseitigen Spulenwindungsbereichen weiter von der Spulenkasten-Bodenwand abgehoben sind als die weiter radial innen liegenden Spulenwindungsbereiche. Die Kühlluft wird hiermit somit durch den bodenseitigen Kühlluft-Eintritt-Ringspalt zugeführt und prallt von dort auf die gestaffelten unteren Kanten der Spulenwindungen, dort wo die größte Erwärmung der Spule durch Wirbelströme im Kupfer durch das erzeugte Magnetfeld entsteht. Durch die keilförmige Ausführung insbesondere der bodenseitigen Spulenträger und die dadurch bedingte Querschnittverengung zum Zentrum hin entsteht an der innersten Spulenwindung die vorteilhafte höchste Luftgeschwindigkeit, d. h. dort wo der größte Kühlbedarf ist. Die Kühlluft strömt dabei durch die Windungsabstände hindurch und kann oberhalb der Magnetspule frei austreten, wobei keine weiteren Staustellen entstehen.

Um die Kühlluftströmung zu dem radial inneren Spulenwindungsbereich weiterfördern kann nach Anspruch 10 ausgehend vom Kühlluft-Eintritt-Ringspalt wenigstens eine etwa bodenparallele Luftleitplatte angeordnet sein. Zweckmäßig werden zwei mit einem Abstand übereinander liegende Luftleitplatten jeweils im Bereich zwischen zwei bodenseitigen Spulenträgern angebracht, wobei die bodennähere Luftleitplatte länger und breiter ausgebildet sein soll. Diese Luftleitplatten können einfach nach Anspruch 11 mittels Abstandssäulen und/oder Distanzringen mit der Spulenkasten-Bodenwand verschraubt sein.

In Anspruch 12 wird vorgeschlagen, dass die Spulenträger Aufnahmebohrungen zur Halterung der Isolierstäbe aufweisen und die bodenseitigen Spulenträger mit der Spulenkasten-Bodenwand insbesondere der Polschuhplatte verschraubt und/oder verklebt sind. Die deckelseitigen Spulenträger sind dagegen radial außen lösbar mit der Deckelwand verschraubt und radial innen über Stützsäulen mit der Spulenkasten-Bodenwand lösbar verbunden. Die Lösbarkeit der deckelseitigen Spulenträger ist wesentlich für eine einfache Montage der Spulenwindungen. Durch Schraub- und/oder Klebeverbindungen zwischen der Spulenkasten-Bodenwand, den Spulenkasten-Seitenwänden und der Spulenkasten-Deckelwand wird ein stabiler Spulenkasten geschaffen, der über weitere Verbindungselement wie beispielsweise über Gewindestangen mit benachbarten Magnetjochteilen verbindbar ist. Eine Polschuhplatte wird durch ihre seitliche Verbindung mit den Spulenkasten-Seitenwänden versteift, so dass sie vorteilhaft nur eine geringe Durchbiegung aufweist, obwohl ggf. im Polschuhbereich eine ausgefräste Vertiefung vorgesehen sein kann.

Eine besonders bevorzugte Kombination der erfindungsgemäßen Anordnung ergibt sich mit einer an sich bekannten Magnetjochform nach Anspruch 13 in der Art einer M-Form als Drei-Schenkelanordnung.

Anhand einer Zeichnung wird die Erfindung näher erläutert.

Es zeigen:
- Fig. 1: eine schematische Schnittansicht durch einen Magnetfeldapplikator,
- Fig. 2: eine schematische Draufsicht auf den Magnetfeldapplikator gemäß Fig. 1,
- Fig. 3: eine schematische Seitenansicht des Magnetfeldapplikators gemäß Fig. 1,
- Fig. 4: eine schematische, perspektivische und vergrößerte Darstellung des Aufbaus quaderförmiger Ferritbausteine,
- Fig. 5: eine schematische, vergrößerte Darstellung der Einzelheit A aus Fig. 1,
- Fig. 6: eine schematische, vergrößerte Draufsicht entsprechend Fig. 5,
- Fig. 7: ein schematischer Querschnitt durch einen oberen Spulenkasten mitsamt zugeordnetem Polschuh gemäß einer alternativen Ausführungsform, und
- Fig. 8: eine schematische Draufsicht auf einen Spulenkasten mit zugeordnetem Polschuh und zugeordneter Magnetspule.

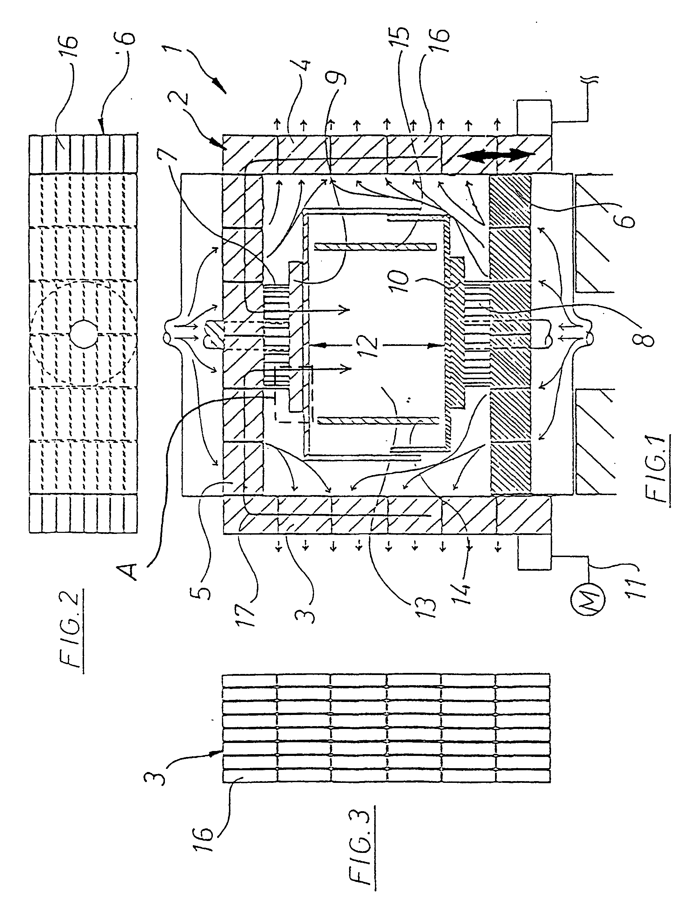

In der Fig. 1 ist schematisch ein Magnetfeldapplikator 1 für die Hyperthermie dargestellt, in den ein zu befeldender Körper, in den eine magnetische oder magnetisierbare Substanz oder Festkörper einbringbar ist, bestrahlbar ist. Als zu befeldender Körper eignet sich insbesondere ein Tumorbereich in einem menschlichen Körper, in dem eine Flüssigkeit mit z. B. magnetischen Nanoteilchen eingebracht ist, wobei der Tumorbereich auf Temperaturwerte von vorzugsweise über ca. 41°C erhitzbar ist.

Der Magnetfeldapplikator 1 umfasst ein Magnetjoch 2, das in einer M-Form als Drei-Schenkel-Anordnung ausgebildet ist und zwei beabstandete, parallele Vertikaljochteile 3, 4 sowie zwei dazwischen angeschlossene Querjochteile 5, 6 aufweist.

Eine Baueinheit aus unterem Querjochteil 6 und diesem zugeordneten unteren Polschuh 8 mit unterem Spulenkasten 10 ist ortsfest angebracht. Demgegenüber kann ein Portal aus den beiden Vertikaljochteilen 3, 4, dem angeschlossenen oberen Querjochteil 5 sowie diesem zugeordneten Polschuh 7 mit oberem Spulenkasten 9 mittels eines hier lediglich schematisch dargestellten, selbsthemmenden Spindelantriebs 11 zur Einstellung der Befeldungsspaltweite des Befeldungsspaltes 12 verstellt werden.

Der Fig. 1 kann weiter entnommen werden, dass der Befeldungsspalt 12 durch Schottwände 14, 15 begrenzt ist, die einen Einschubraum 13 umgrenzen. Die Schottwände 14, 15 können dabei gegeneinander vertikal verstellbar sein.

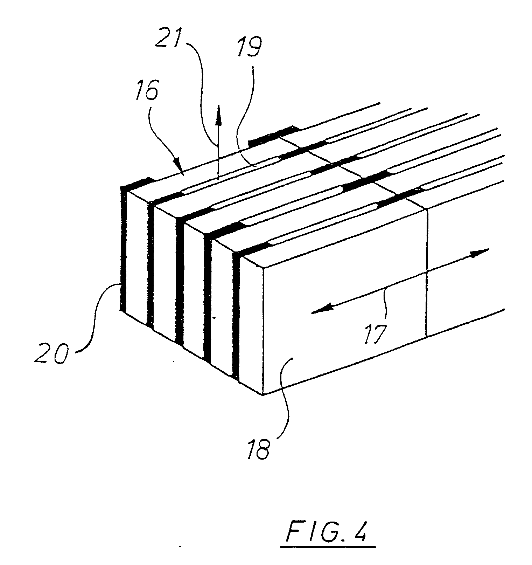

Wie dies aus den Fig. 2 und 3 ersichtlich ist, die eine Seitenansicht bzw. eine Draufsicht auf das Magnetjoch 2 zeigen, ist das Magnetjoch 2 aus quaderförmigen Ferritbausteinen 16 zusammengesetzt, deren Oberflächen vorzugsweise von Sinterschichten befreit und jeweils plan-parallel geschliffen sind. Diese quaderförmigen Ferritbausteine 16 sind wiederum, wie dies aus der Fig. 4 ersichtlich ist, aus aneinandergereihten, im Magnetjoch 2 längs der Magnetflussrichtung 17 ausgerichteten Ferritplatten 18 aufgebaut.

Diese Ferritplatten 18 sind quer zur Magnetflussrichtung 17 durch Isolations/Kühlungs-Spalte 19 voneinander getrennt (Fig. 4). In diese Isolations/Kühlungs-Spalte 19 sind in Seitenbereichen Kunststoffseparatoren 20 eingefügt, wobei die Ferritplatten 20 über diese Kunststoffseparatoren 20 zu den quaderförmigen Ferritbausteinen 16 als Jochelemente verklebt sind. Durch die Isolations/Kühlungs-Spalte 19 kann zur Kühlung des Magnetjochs 2 Kühlluft geleitet werden, wie dies in der Fig. 4 mit dem Pfeil 21 schematisch dargestellt ist.

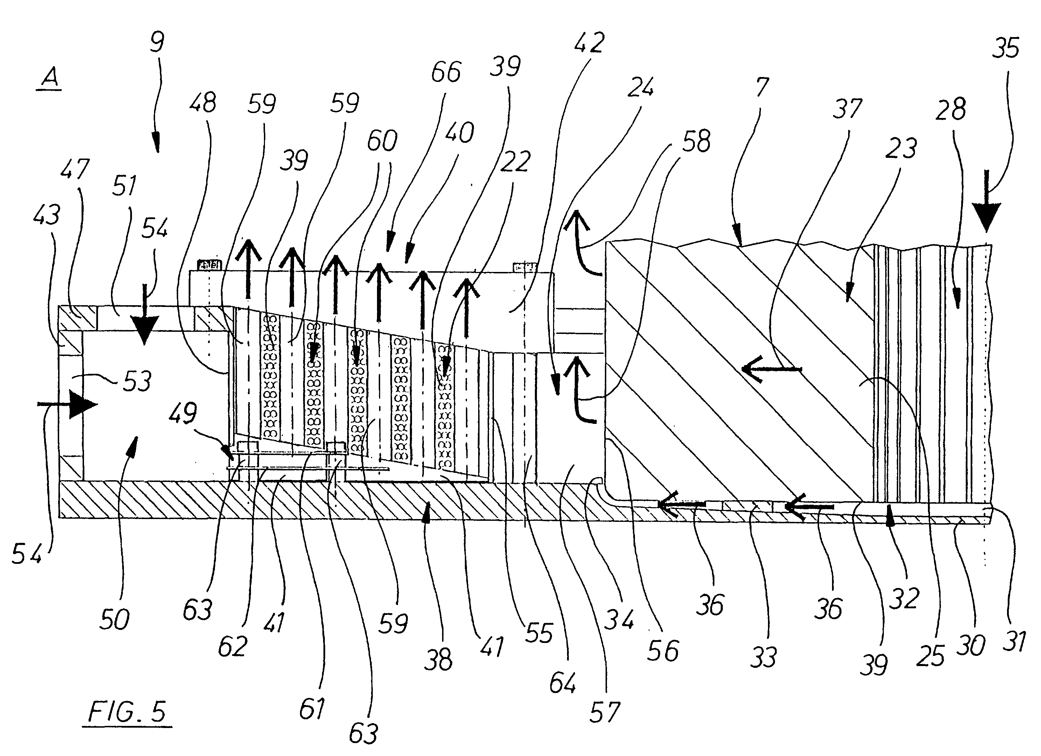

Wie dies insbesondere aus der Fig. 5, die eine vergrößerte Detailansicht der Einzelheit A aus Fig. 1 zeigt, ersichtlich ist, ist zur Erzeugung eines Magnetwechselfeldes dem Polschuh 7 eine Magnetspule 22 zugeordnet, die als Scheibenspule mit schneckenförmig verlaufenden Spulenwindungen 39 ausgebildet ist und jeweils ein Polschuhende des Polschuhs 7 mit einem dazwischenliegenden, umlaufenden Magnetspule/Polschuh-Spalt 24 ringförmig umfasst.

Entsprechend identisch ist auch der Aufbau am unteren Polschuh 8 mit unteren Spulenkasten 10 ausgebildet, wobei hier nachfolgend aus Gründen der Einfachheit der genaue Aufbau stets lediglich in Verbindung mit dem oberen Polschuh 7 und dem oberen Spulenkasten 9 sowie in Verbindung mit der Magnetspule 22 näher erläutert wird.

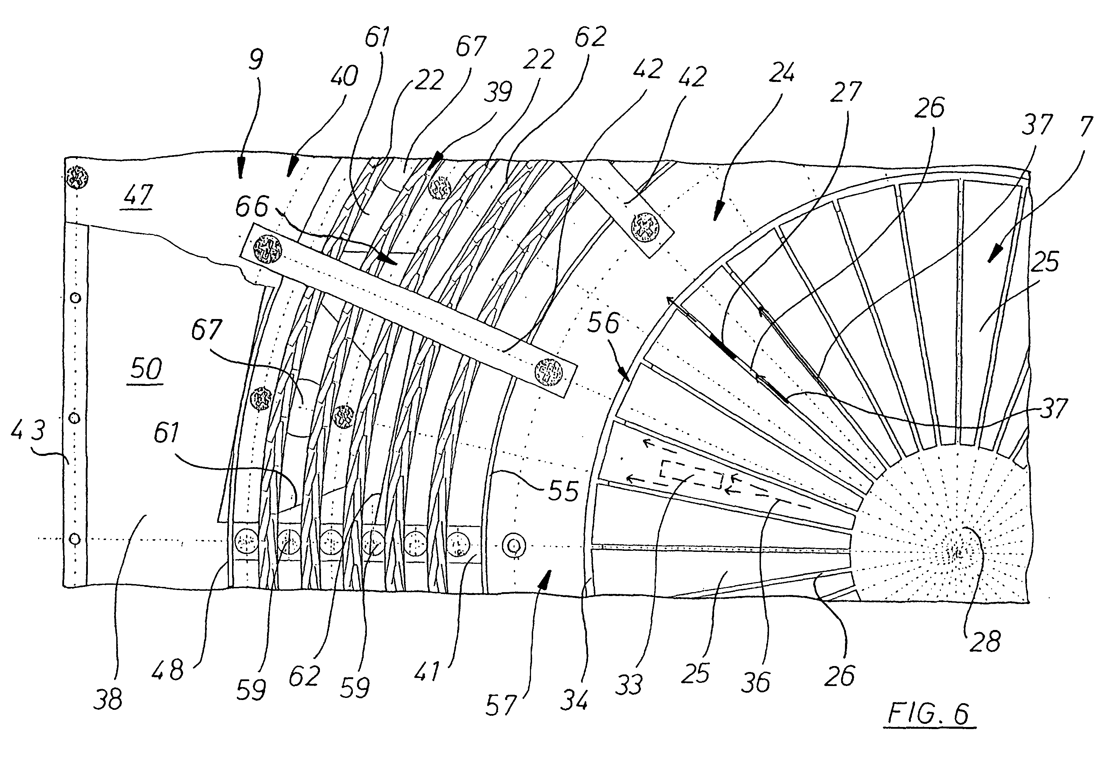

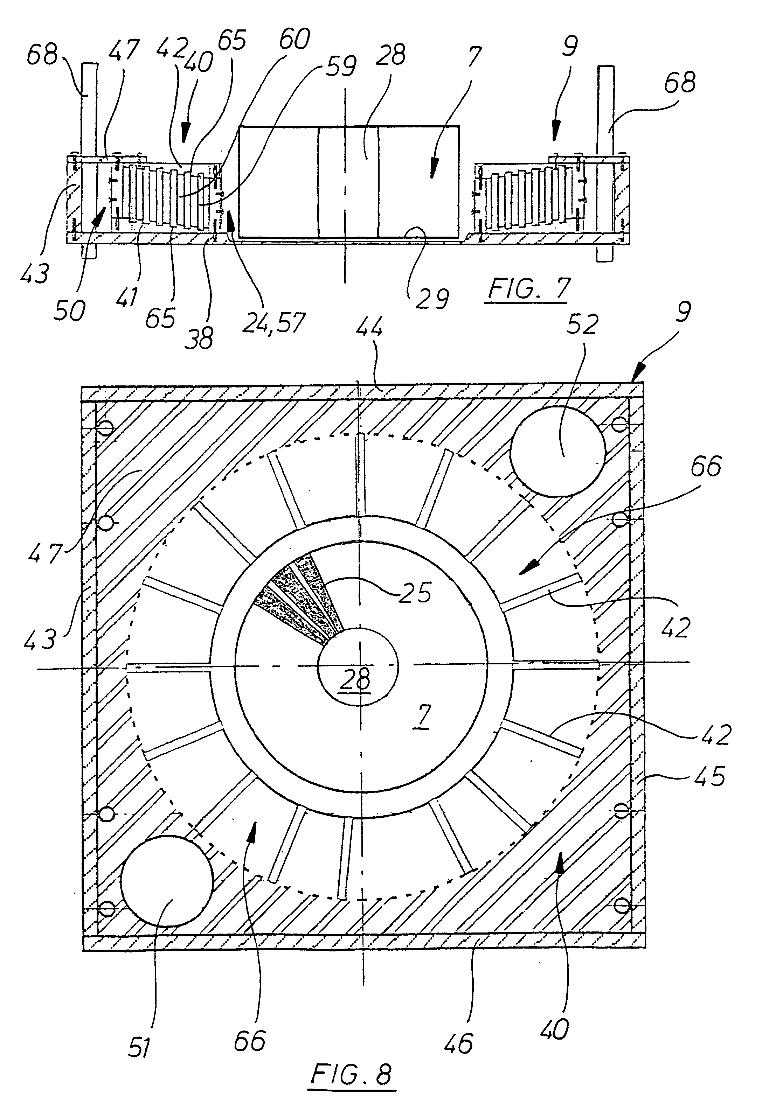

Die Polschuhe 7, 8 sind in einer Draufsicht kreisrund ausgebildet und aus entsprechend bearbeiteten, in der Draufsicht keilförmigen Ferritbausteinen zusammengesetzt, wie dies insbesondere aus der Fig. 6, die eine vergrößerte Draufsicht der Darstellung gemäß Fig. 5 zeigt, und aus der Fig. 8 ersichtlich ist, die eine Draufsicht auf den oberen Spulenkasten 9 mitsamt oberen Polschuh 7 zeigt.

Zwischen die keilförmigen Ferritbausteine 25 sind zur Bildung von Isolations/Kühlungs-Spalten 26 jeweils Separatoren 27, von denen in der Darstellung der Fig. 6 lediglich einer schematisch und beispielhaft eingezeichnet ist, eingefügt, über die benachbarte Ferritbausteine 25 miteinander zu dem Polschuh 7 verklebt sind. Der Polschuh 7 umfasst ferner eine axiale, rohrförmige Aussparung 28 zur Bildung eines rohrförmigen Polschuhs 7.

Wie dies insbesondere aus der Fig. 5 ersichtlich ist, ist eine Polschuhendfläche durch eine Polschuhplatte 30 abgedeckt, wobei die Polschuhplatte 30 die zugeordnete Polschuhendfläche 29 seitlich umlaufend überragt und im Bereich der Polschuhendflächen 29 eine Vertiefung 31 mit geringerer Materialstärke als im sich daran anschließenden Bereich aufweist, in die die Polschuhendfläche 29 eintaucht. Zwischen der Polschuhendfläche 29 und der Polschuhplatte 30 sind zur Bildung von Isolations/Kühlungs-Spalten 32 Separatoren 33 eingefügt, von denen in den Darstellungen der Figuren 5 und 6 lediglich einer schematisch und beispielhaft eingezeichnet ist, eingefügt. Die umlaufende Kante der Polschuhendfläche 29 ist ebenso wie die zugeordnete Kante der Vertiefung 31 abgerundet, so dass die lsolations/Kühlungs-Spalte 32 in einem Ringspalt 34 als Kühlluftaustritt enden.

Wie dies in der Fig. 5 durch die Pfeile dargestellt ist, kann über die Aussparung 28 Kühlluft entsprechend dem Pfeil 35 dem Polschuh 7 zugeführt werden, wobei diese Kühlluft dann sowohl über die vertiefungsseitigen Isolations/Kühlungs-Spalte 32 und den Ringspalt 34 entsprechend den Pfeilen 36 als auch über die Isolations/Kühlungs-Spalte 26 entsprechend den Pfeilen 37 zwischen den einzelnen Ferritbausteinen 25 hindurchströmen kann, wie dies aus den Fig. 5 und 6 ersichtlich ist.

Wie dies insbesondere aus der Fig. 5 in Verbindung mit der Fig. 8 ersichtlich ist, ist der obere Spulenkasten 9 quaderförmig ausgebildet, wobei der die zugeordnete Polschuhendfläche 29 seitlich umlaufend überragende Bereich der Polschuhplatte 30 eine befeldungsspaltseitige Spulenkasten-Bodenwand 38 ausbildet.

An die Spulenkasten-Bodenwand 38 schließen sich, wie dies insbesondere aus der Fig. 8 ersichtlich ist, Spulenkasten-Seitenwände 43, 44, 45, 46 an. Auf diese Spulenkasten-Seitenwände 43, 44, 45, 46 ist, wie dies insbesondere aus der Fig. 5 in Verbindung mit der Fig. 8 ersichtlich ist, eine Spulenkasten-Deckelwand 47 aufgesetzt. Ferner umfasst der Spulenkasten 9 auch noch eine die Magnetspule 22 radial außen umgebende, dünnwandige Windkasten-Innenwand, welche von der Spulenkasten-Bodenwand 38 mit einem Kühlluft-Eintrittringspalt 49 beabstandet ist. Die Spulenkasten-Bodenwand 38, die Spulenkasten-Seitenwände 43, 44, 45 und 46, die Spulenkasten-Deckelwand 47 sowie die Windkasten-Innenwand bilden einen umlaufenden Windkasten 50 auf, wobei die Kühlluftzufuhr zum Windkasten 50 hier beispielhaft über deckelwandseitige Einströmöffnungen 51, 52 und über seitenwandseitige Einströmöffnungen 53 erfolgen kann. An diese Kühlluft-Einströmöffnungen 51, 52, 53 kann eine hier nicht dargestellte Kühlluftpumpe angeschlossen werden, über die Kühlluft entsprechend der Pfeile 54 in den Windkasten 50 eingeblasen werden kann.

Die Magnetspule 22 ist ferner radial innen von einer Luftführungswand 55 umgeben, wodurch zwischen dieser und einer benachbarten Polschuhwand 56 ein Ringspalt 57 zum Austritt der Polschuh-Kühlluft entsprechend der Pfeile 58 erfolgen kann.

Die Magnetspule 22 weist eine Stützstruktur 40 für die Spulenwindungen 39 auf, die im Bereich der Magnetspule 22 stegförmige bodenseitige Spulenträger 41 und darüber liegend angeordnete, stegförmige deckelseitige Spulenträger 42 als zugeordnete Spulenträgerpaare aufweist. Wie dies insbesondere aus der Fig. 6 und der Fig. 8 ersichtlich ist, sind die einander zugeordneten Spulenträgerpaare aus bodenseitigem Spulenträger 41 und deckelseitigem Spulenträger 42 jeweils radial strahlenförmig und von dem benachbarten Spulenträgerpaar winkelversetzt beabstandet angeordnet.

In der Fig. 6 ist in einer unteren Bildebenenbereich eines der Spulenträgerpaare ohne den deckelseitigen Spulenträger 42 dargestellt. Aus Fig. 5 ist ersichtlich, dass paarweise einander zugeordnete Spulenträger 41, 42 jeweils durch radial beabstandete und etwa axial ausgerichtete Isolierstäbe 59 verbunden sind. Diese Isolierstäbe 59 sind z. B. als Keramik-Rundstäbe ausgebildet, wobei zwischen den Isolierstäben 59 Aufnahmefächer 60 gebildet sind, in denen die schneckenförmig verlaufenden Spulenwindungen 39 aufgenommen und gehalten sind.

Die Spulenträger 41, 42 sind, wie dies insbesondere aus der Fig. 5 ersichtlich ist, jeweils keilförmig ausgebildet, so dass die radial äußeren Spulenwindungen mit ihren bodenseitigen Spulenwindungsbereichen weiter von der Spulenkasten-Bodenwand 38 abgehoben sind als die weiter radial innenliegenden Spulenwindungsbereiche.

Ausgehend vom Kühlluft-Eintrittringspalt 49 im Keilbereich zwischen den bodenseitigen Spulenträgern 41 erstrecken sich in etwa bodenparallele Luftleitplatten 61, 62, wobei jeweils die bodennähere Luftleitplatte 62 länger und breiter ausgebildet ist als die jeweils darüber liegende zugeordnete Luftleitplatte 61. Die Luftleitplatten 61, 62 sind über Abstandssäulen 63 mit der Spulenkasten-Bodenwand 38 verbunden.

Wie dies insbesondere aus der Fig. 5 und der Fig. 6 ersichtlich ist, sind die deckelseitigen Spulenträger 42 radial außen lösbar mit der Deckelwand 47 verschraubt und radial innen mit auf der Spulenkasten-Bodenwand 38 angebrachten Stützsäulen 64 ebenfalls lösbar verschraubt. In der Fig. 7, die einen schematischen Querschnitt durch den gesamten Spulenkasten 9 zeigt, ist eine alternative Ausführungsform gezeigt, bei der die deckelseitigen Spulenträger von unten her mit der Deckelwand 47 verschraubt sind. Zudem sind hier noch randseitig Gewindestangen 68 angeordnet über die eine Verbindung mit anderen Bauteilen möglich ist. Ansonsten entspricht der Aufbau demjenigen, wie er in der vergrößerten Detaildarstellung der Fig. 5 gezeigt ist.

Die bodenseitigen Spulenträger 41 sind mit der Spulenkasten-Bodenwand 38 verschraubt und/oder verklebt.

Wie dies insbesondere aus der Fig. 7 ersichtlich ist, können die Spulenträger 41, 42 jeweils Aufnahmebohrungen 65 aufweisen, in die die Isolierstäbe 59 eingesteckt und gehalten sind.

Wie dies insbesondere aus der Fig. 5 ersichtlich ist, wird somit die Kühlluft über den z. B. als Druckkammer fungierenden Windkasten 50 über den bodenseitigen Kühlluft-Eintrittringspalt 49 im Bereich der unteren Spulenwindungsbereiche zugeführt, da dort die größte Erwärmung der Magnetspule entsteht. Dadurch, dass die radial äußeren Spulenwindungen mit ihren bodenseitigen Spulenwindungsbereichen weiter von der Spulenkasten-Bodenwand 38 abgehoben sind als die weiter radial innen liegenden Spulenwindungsbereiche prallt die über den Kühlluft-Eintrittringspalt 49 zugeführte Kühlluft somit direkt auf die dort gestaffelten unteren Kanten der Spulenwindungen 39, dort wo die größte Erwärmung der Spule durch Wirbelströme im Kupfer durch das erzeugte Magnetfeld entsteht. Durch die keilförmige Ausführung und der dadurch bedingten Querschnittsverengung zum Zentrum hin entsteht in diesem bodenseitigen Bereich an der innersten Spulenwindung die vorteilhaft höchste Luftgeschwindigkeit, d. h. dort wo der größte Kühlbedarf ist. Die Kühlluft strömt dabei durch die Windungsabstände hindurch und kann oberhalb der Magnetspule 22 im Bereich 66 der nicht abgedeckten Spulenwindungen (Fig. 8) frei austreten, so dass diese Bereiche 66 die Kühlluft-Ausströmöffnungen bilden. Insbesondere durch die Luftleitfläche 61, 62 wird die Kühlluftströmung zu dem radial inneren Spulenwindungsbereich weiter gefördert, da unmittelbar vom Kühlluft-Eintrittringspalt ausgehend Teilluftströme abgezweigt werden, die erst in diesem radial inneren Spulenwindungsbereich nach oben strömen können.

In der Luftleitplatte 61 kann, wie dies aus der Fig. 6 ersichtlich ist, ferner eine Durchströmöffnung 67 nach oben hin ausgebildet sein. Damit wird erreicht, dass in Verbindung mit den Luftleitblechen 61, 62 mehr Luft zu den äußeren Windungen gelangen kann.

## Patentansprüche

1. Magnetspulenanordnung eines Magnetfeldapplikators (1) zur Aufheizung von magnetischen oder magnetisierbaren Substanzen oder Festkörpern in biologischem Gewebe,
mit einem kühlbaren Magnetjoch (2) mit zwei gegenüberliegenden, durch einen Befeldungsspalt beabstandeten Polschuhen (7, 8) am Magnetjoch (2), wobei zur Erzeugung eines Magnetwechselfeldes zwei jeweils einem Polschuh zugeordnete Magnetspulen (22) vorgesehen sind, die als Scheibenspulen mit schneckenförmig verlaufenden Spulenwindungen ausgebildet sind und jeweils das Polschuhende des zugeordneten Polschuhs (7, 8) mit einem dazwischenliegenden umlaufenden Magnetspule/Polschuh-Spalt (24) ringförmig umfassen,
**dadurch gekennzeichnet,**
**dass** jeweils die Magnetspule (22) zur Umströmung ihrer Windungen mit Kühlluft in einem den zugeordneten Polschuh (7, 8) ringförmig umfassenden Spulenkasten (9, 10) angebracht ist, und
**dass** der Spulenkasten (9, 10) wenigstens eine Kühlluft-Einströmöffnung (51, 52, 53) zum Anschluss einer Kühlluftpumpe und wenigstens eine Kühlluft-Ausströmöffnung (57, 66) aufweist.

2. Magnetspulenanordnung nach Anspruch 1, **dadurch gekennzeichnet,**
**dass** das Magnetjoch (2) aus quaderförmigen Ferritbausteinen (16) zusammengesetzt ist, deren Flächen gegebenenfalls von Sinterschichten befreit und plan-parallel geschliffen sind, wobei die quaderförmigen Ferritbausteine (16) aus aneinandergereihten, im Magnetjoch (2) längs des Magnetflusses (17) ausgerichteten Ferritplatten bestehen, die quer zum Magnetfluss (17) durch Isolations-/Kühlungs-Spalte (19) voneinander getrennt sind, durch die Kühlluft (21) leitbar ist und die längs des Magnetflusses (17) über einen nur schmalen Anlagespalt aneinander liegen und in die Isolations-/Kühlungs-Spalte (19) vorzugsweise in Seitenbereichen Separatoren (20) aus Kunststoff eingefügt sind, über die die Ferritplatten zu Ferritbausteinen (16) als Jochelemente verklebt sind,
**dass** runde Polschuhe (7, 8) aus entsprechend bearbeiteten, in der Draufsicht keilförmigen Ferritbausteinen (25) zusammengesetzt sind, wobei
zwischen die keilförmigen Ferritbausteine (25) zur Bildung von Isolations-/Kühlungs-Spalten Separatoren (27) eingefügt sind, über die benachbarte Ferritbausteine (25) miteinander zu einem Polschuh (7) verklebt sind, und
eine axiale, rohrförmige Aussparung (28) zur Bildung jeweils eines rohrförmigen Polschuhs (7) vorgesehen ist und durch die Aussparung (28) Kühlluft einleitbar ist.

3. Magnetspulenanordnung nach Anspruch 1 oder Anspruch 2, **dadurch gekennzeichnet,**
**dass** die Polschuhendflächen (29) jeweils durch eine Polschuhplatte (30) abgedeckt sind und die Polschuhplatte (30) unter Bildung eines Bereichs einer befeldungsspaltseitigen Spulenkasten-Bodenwand (38) die jeweils zugeordnete Polschuhendfläche (29) seitlich umlaufend überragt, und
**dass** zwischen einer Polschuhendfläche (29) und der Polschuhplatte (30) zur Bildung von Isolations-/Kühlungs-Spalten (32) Separatoren (33) eingefügt sind.

4. Magnetspulenanordnung nach Anspruch 3, **dadurch gekennzeichnet, dass** die Polschuhplatte (30) im Bereich der Polschuhendfläche (29) eine Vertiefung (31) mit geringerer Materialstärke als im Bereich der Spulenkasten-Bodenwand (38) aufweist, in die die Polschuhendfläche (29) eintaucht, wobei die umlaufende Kante der Polschuhendfläche (29) abgerundet ist und dort die Isolations-/Kühlungs-Spalten (32) in einem Ringspalt (34) als Kühlluftaustritt enden.

5. Magnetspulenanordnung nach einem der Ansprüche 1 bis Anspruch 3, **dadurch gekennzeichnet,**
**dass** die Polschuhe (7, 8) in einer Draufsicht kreisrund ausgebildet sind und mit gegeneinander weisenden, parallel ausgerichteten Polschuhkreisflächen als Polschuhendflächen im Abstand des Befeldungsspalts (12) gegenüberliegen,
**dass** die Magnetspulen (22) entsprechend kreisringförmig ausgebildet sind,
**dass** ein Spulenkasten (9, 10) quaderförmig das zugeordnete Polschuhende und die dieses Polschuhende umgebende Magnetspule (22) umfasst, und
**dass** die wenigstens eine Kühlluft-Einströmöffnung (51, 52, 53) in einer Spulenkasten-Seitenwand (43) und/oder in wenigstens einem Eckbereich einer Spulenkasten-Deckelwand (47) angeordnet ist.

6. Magnetspulenanordnung nach einem der Ansprüche 1 bis Anspruch 5, **dadurch gekennzeichnet,**
**dass** die Magnetspule (22) eine Stützstruktur (40) für die Spulenwindungen (39) aufweist, dergestalt
**dass** im Bereich der Magnetspule (22) stegförmige bodenseitige Spulenträger (41) und darüber liegend angeordnete, stegförmige deckelseitige Spulenträger (42) als zugeordnete Spulenträgerpaare (41, 42) vorgesehen sind, die radial strahlenförmig und gegeneinander winkelversetzt angeordnet sind,
**dass** paarweise zugeordnete Spulenträger (41, 42) jeweils durch radial beabstandete und etwa axial ausgerichtete Isolierstäbe (59) verbunden sind, und
**dass dadurch** zwischen den Isolierstäben (59) Aufnahmefächer (60) gebildet sind, in denen die schneckenförmig verlaufenden Spulenwindungen (39) aufgenommen und gehalten sind, dergestalt,
**dass** durch die Steghöhe der bodenseitigen Spulenträger (41) die Spulenwindungen (39) unter Bildung eines radial äußeren Kühlluft-Eintrittringspalts (49) gegenüber der Spulenkasten-Bodenwand (38) abgehoben sind, wobei Kühlluft weiter durch die von den Isolierstäben (59) bestimmten Zwischenspalte führbar ist und die Bereiche (66) zwischen den deckelseitigen Spulenträgern (42) über den nicht abgedeckten Spulenwindungen (39) Kühlluft-Ausströmöffnungen bilden.

7. Magnetspulenanordnung nach Anspruch 6, **dadurch gekennzeichnet, dass** die Isolierstäbe (59) als Keramik-Rundstäbe ausgebildet sind.

8. Magnetspulenanordnung nach Anspruch 6 oder Anspruch 7, **dadurch gekennzeichnet,**
**dass** die Spulenkasten-Bodenwand (38), die Spulenkasten-Seitenwände (43, 44, 45, 46), die Spulenkasten-Deckelwand (47) sowie eine die Magnetspule (22) radial außen umgebende, dünnwandige Windkasteninnenwand (48), welche von der Spulenkasten-Bodenwand (38) mit dem Kühlluft-Eintrittringspalt (49) beabstandet ist, einen umlaufenden Windkasten (50) bilden und
**dass** die Magnetspule (22) radial innen von einer Luftführungswand (55) umgeben ist, wodurch zwischen dieser Luftführungswand (55) und einer benachbarten Polschuhwand (56) ein Ringspalt (24, 57) zum Austritt der Polschuh-Kühlluft hergestellt ist.

9. Magnetspulenanordnung nach einem der Ansprüche 6 bis Anspruch 8, **dadurch gekennzeichnet, dass** die Spulenträger (41, 42) keilförmig ausgebildet sind, dergestalt, dass die radial äußeren Spulenwindungen (39) mit ihren bodenseitigen Spulenwindungsbereichen weiter von der Spulenkasten-Bodenwand (38) abgehoben sind als die weiter radial innen liegenden Spulenwindungsbereiche.

10. Magnetspulenanordnung nach Anspruch 9, **dadurch gekennzeichnet, dass** sich ausgehend vom Kühlluft-Eintrittringspalt (49) im Keilbereich zwischen den bodenseitigen Spulenträgern (41) etwa bodenparallele Luftleitplatten (61, 62) erstrecken, wobei jeweils eine bodennähere Luftleitplatte (62) länger und breiter ausgebildet ist als eine jeweils darüber liegende zugeordnete Luftleitplatte (61).

11. Magnetspulenanordnung nach Anspruch 10, **dadurch gekennzeichnet, dass** die Luftleitplatten (61, 62) über Abstandssäulen (63) und/oder Distanzringe mit der Spulenkasten-Bodenwand (38) verbunden sind.

12. Magnetspulenanordnung nach einem der Ansprüche 6 bis Anspruch 11, **dadurch gekennzeichnet,**
**dass** die Spulenträger Aufnahmebohrungen (65) aufweisen, in die die Isolierstäbe (59) eingesteckt und gehalten sind,
**dass** die bodenseitigen Spulenträger (41) mit der Spulenkasten-Bodenwand (38) verschraubt und/oder verklebt sind,
**dass** die deckelseitigen Spulenträger (42) radial außen lösbar mit der Deckelwand (47) verschraubt sind und radial innen mit auf der Spulenkasten-Bodenwand (38) angebrachten Stützsäulen (64) lösbar verschraubt sind,
**dass** durch Schraub- und/oder Klebeverbindungen zwischen der Spulenkasten-Bodenwand (38), den Spulenkasten-Seitenwänden (43, 44, 45, 46) und der Spulenkasten-Deckelwand (47) ein stabiler Spulenkasten-Aufbau vorliegt, der über Verbindungselemente mit einem benachbarten Magnetjochteil verbindbar ist.

13. Magnetspulenanordnung nach einem der Ansprüche 1 bis 12, **dadurch gekennzeichnet, dass** das Magnetjoch (2) in einer M-Form als Drei-Schenkel-Anordnung ausgebildet ist, mit zwei beabstandeten, parallelen Vertikaljochteilen (3, 4) gleicher Geometrie und mit zwei dazwischen angeschlossenen Querjochteilen (5, 6) mit jeweils in deren Mitte angeordneten und aufeinander zugerichteten Polschuhen (7, 8), wovon wenigstens ein Querjochteil (5, 6) mit angeschlossenem Polschuh (7, 8) und zugeordneter Magnetspule (22) als Baueinheit relativ zum anderen Querjochteil (5, 6) zur Einstellung der Befeldungsspaltweite (12) verstellbar ist.

## Claims

1. Magnetic coil arrangement of a magnetic field applicator (1) for heating magnetic or magnetizable substances or solid bodies in biological tissue,
comprising a coolable magnetic yoke (2) with two pole shoes (7, 8) on the magnetic yoke (2) facing each other and separated by a gap defining the magnetic field exposure volume, two magnetic coils (22) respectively assigned to a pole shoe being provided for the production of an alternating magnetic field, which magnetic coils are formed as disc coils with helicoidally extending coil windings and in each case annularly enclose the pole shoe end of the assigned pole shoe (7, 8) with a peripheral magnetic-coil/pole-shoe gap (24) lying in between,
**characterized**
**in that**, for cooling air to flow around its windings, the magnetic coil (22) is in each case fitted in a coil casing (9, 10) annularly enclosing the assigned pole shoe (7, 8), and
**in that** the coil casing (9, 10) has at least one cooling air inflow opening (51, 52, 53) for the connection of a cooling air pump and at least one cooling air outflow opening (57, 66).

2. Magnetic coil arrangement according to Claim 1, **characterized**
**in that** the magnetic yoke (2) is composed of cuboidal ferrite segments (16), the surfaces of which are ground free of any sintering layers and such that they are plane-parallel, the cuboidal ferrite segments (16) comprising ferrite plates which are aligned in rows along the magnetic flow (17) in the magnetic yoke (2) and are separated from one another transversely to the magnetic flow (17) by isolating/cooling gaps (19), through which cooling air (21) can be passed and which lie adjacent to one another along the magnetic flow (17) with only a narrow abutment gap, and separators (20) of plastic are inserted into the isolating/cooling gaps (19), preferably in side regions, via which separators the ferrite plates are adhesively bonded to form ferrite segments (16) as yoke elements,
**in that** round pole shoes (7, 8) are composed of correspondingly machined ferrite segments (25) which are wedge-shaped in plan view,
separators (27) via which adjacent ferrite segments (25) are adhesively bonded to one another to form a pole shoe (7) being inserted between the wedge-shaped ferrite segments (25) to form isolating/cooling gaps, and
an axial, tubular opening (28) being provided for respectively forming a tubular pole shoe (7) and it being possible for cooling air to be passed through the opening (28).

3. Magnetic coil arrangement according to Claim 1 or Claim 2, **characterized**
**in that** the pole shoe end faces (29) are respectively covered by a pole shoe plate (30) and the pole shoe plate (30) protrudes laterally beyond the periphery of the respectively assigned pole shoe end face (29), thereby forming a region of a coil casing bottom wall (38) on the side of the gap defining the magnetic field exposure volume, and
**in that**, to form isolating/cooling gaps (32), separators (33) are inserted between a pole shoe end face (29) and the pole shoe plate (30).

4. Magnetic coil arrangement according to Claim 3, **characterized in that** the pole shoe plate (30) has in the region of the pole shoe end face (29) a depression (31) with a smaller material thickness than in the region of the coil casing bottom wall (38), into which depression the pole shoe end face (29) enters, the peripheral edge of the pole shoe end face (29) being rounded off and the isolating/cooling gaps (32) ending there in an annular gap (34) as a cooling air outlet.

5. Magnetic coil arrangement according to one of Claims 1 to 3, **characterized**
**in that** the pole shoes (7, 8) are of a circular form in plan view and lie with mutually facing, parallel aligned circular pole shoe surfaces as pole shoe end faces spaced apart by the gap defining the magnetic field exposure volume (12),
**in that** the magnetic coils (22) are of a correspondingly circular ring-shaped form,
**in that** a coil casing (9, 10) encloses in a cuboidal manner the assigned pole shoe end and the magnetic coil (22) surrounding this pole shoe end, and
**in that** the at least one cooling air inflow opening (51, 52, 53) is arranged in a coil casing side wall (43) and/or in at least one corner region of a coil casing top wall (47).

6. Magnetic coil arrangement according to one of Claims 1 to 5, **characterized**
**in that** the magnetic coil (22) has a supporting structure (40) for the coil windings (39), in such a way
that web-shaped bottom coil formers (41) and, arranged lying above them, web-shaped top coil formers (42) are provided in the region of the magnetic coil (22) as assigned pairs of coil formers (41, 42), which are arranged in a radially radiating form and angularly offset with respect to one another,
that coil formers (41, 42) assigned in pairs are respectively connected by radially spaced-apart and approximately axially aligned insulating bars (59), and
that, as a result, receiving compartments (60) in which the helicoidally extending coil windings (39) are accommodated and held are formed between the insulating bars (59), in such a way
that the coil windings (39) are raised with respect to the coil casing bottom wall (38) by the web height of the bottom coil formers (41), thereby forming a radially outer annular cooling air inlet gap (49), it being possible for cooling air to be conducted further through the intermediate gaps determined by the insulating bars (59), and the regions (66) between the top coil formers (42) over the noncovered coil windings (39) forming cooling air outflow openings.

7. Magnetic coil arrangement according to Claim 6, **characterized in that** the insulating bars (59) are formed as round ceramic bars.

8. Magnetic coil arrangement according to Claim 6 or Claim 7, **characterized**
**in that** the coil casing bottom wall (38), the coil casing side walls (43, 44, 45, 46), the coil casing top wall (47) and a thin-walled wind box inner wall (48), which surrounds the magnetic coil (22) radially on the outside and is spaced apart from the coil casing bottom wall (38) with the annular cooling air inlet gap (49), form a peripheral wind box (50) and
**in that** the magnetic coil (22) is surrounded radially on the inside by an air conducting wall (55), whereby an annular gap (24, 57) for the outlet of the pole shoe cooling air is produced between this air conducting wall (55) and an adjacent pole shoe wall (56).

9. Magnetic coil arrangement according to one of Claims 6 to 8, **characterized in that** the coil formers (41, 42) are formed in a wedge-shaped manner in such a way that the radially outer coil windings (39) are raised further from the coil casing bottom wall (38) with their bottom coil winding regions than the coil winding regions lying further radially inward.

10. Magnetic coil arrangement according to Claim 9, **characterized in that**, starting from the annular cooling air inlet gap (49), air baffle plates (61, 62) approximately parallel to the bottom extend in the wedge region between the bottom coil formers (41), an air baffle plate (62) that is closer to the bottom respectively being made longer and wider than an air baffle plate (61) respectively assigned to lie above it.

11. Magnetic coil arrangement according to Claim 10, **characterized in that** the air baffle plates (61, 62) are connected to the coil casing bottom wall (38) by means of spacing pillars (63) and/or spacer rings.

12. Magnetic coil arrangement according to one of Claims 6 to 11, **characterized**
**in that** the coil formers have receiving bores (65), in which the insulating bars (59) are inserted and held,
**in that** the bottom coil formers (41) are screwed and/or adhesively bonded to the coil casing bottom wall (38),
**in that** the top coil formers (42) are releasably screwed radially on the outside to the top wall (47) and releasably screwed radially on the inside to supporting pillars (64) attached on the coil casing bottom wall (38),
**in that** screwed and/or adhesively bonded connections between the coil casing bottom wall (38), the coil casing side walls (43, 44, 45, 46) and the coil casing top wall (47) provide a stable coil casing construction which can be connected to an adjacent magnetic yoke part by means of connecting elements.

13. Magnetic coil arrangement according to one of Claims 1 to 12, **characterized in that** the magnetic yoke (2) is formed in an M shape as a three-leg arrangement, with two spaced-apart, parallel vertical yoke parts (3, 4) of the same geometry and with two transverse yoke parts (5, 6) connected in between, with pole shoes (7, 8) arranged in each case in the centre thereof and directed at one another, of which at least one transverse yoke part (5, 6) with connected pole shoe (7, 8) and assigned magnetic coil (22) is adjustable as a structural unit in relation to the other transverse yoke part (5, 6) for setting the gap width defining the magnetic field exposure volume (12).

## Revendications

1. Agencement de bobine magnétique d'un applicateur de champ magnétique (1) pour chauffer des substances magnétiques ou magnétisables, ou bien des solides, dans un tissu biologique,
avec une culasse magnétique (2) susceptible d'être refroidie, comprenant deux masses polaires (7, 8) placées à l'opposé l'une de l'autre, espacées de la valeur d'un entrefer d'exposition à un champ, sur la culasse magnétique (2), sachant que, pour produire un champ magnétique alternatif, sont prévues deux bobines magnétiques (2) associées à une masse polaire, bobines magnétiques réalisées respectivement sous la forme de bobines en forme de disque, avec des enroulements de bobine s'étendant en forme d'hélice, et entourant de manière cumulaire respectivement l'extrémité de la masse polaire de la masse polaire (7, 8) associée, avec un intervalle bobine magnétique/masse polaire (24) de pourtour, intermédiaire,
**caractérisé en ce que**
chaque bobine magnétique (22), pour assurer un écoulement de balayage de ses enroulements avec de l'air de refroidissement, est montée dans un caisson à bobine (9, 10), entourant en forme d'anneau la masse polaire (7, 8) associée, et
**en ce que** le caisson de bobine (9, 10) présente au moins une ouverture d'entrée d'écoulement d'air de refroidissement (51, 52, 53), pour le raccordement d'une pompe d'air de refroidissement, et au moins une ouverture de sortie d'écoulement d'air de refroidissement (57, 66).

2. Agencement de bobine magnétique selon la revendication 1, **caractérisé en ce que** la culasse magnétique (2) est composée de composants en ferrite (16) à forme parallélépipédique, dont les faces sont, le cas échéant, débarrassées des couches frittées, et sont meulées à une forme plan-parallèle, sachant que les composants en ferrite (16) à forme parallélépipédique sont formés de plaques en ferrite alignées les unes derrière les autres, orientées dans la culasse magnétique (2), le long du flux magnétique (17), et séparées les unes des autres, transversalement par rapport au flux magnétique (17), par des entrefers isolation/refroidissement (19), à travers lesquels l'air de refroidissement (21) peut être guidé et situés les uns sur les autres le long du flux magnétique (17), sur un intervalle d'appui seulement étroit et, dans les entrefers isolation/refroidissement (19), étant introduits, de préférence dans les zones latérales, des séparateurs (20) en matière synthétique, par lesquels les plaques en ferrite sont collées pour former des composants en ferrite (16) se présentant sous la forme d'éléments de culasse,
**en ce que** des masses polaires (7, 8) rondes sont composées de composants en ferrite (25), usinés de manière correspondante et présentant en vue de dessus une allure cunéiforme, où
entre les composants en ferrite (25) cunéiformes, pour former des séparateurs d'entrefer isolation/refroidissement, sont introduits des séparateurs (27), par l'intermédiaire desquels les composants en ferrite (25) voisins sont collés ensemble pour former une masse polaire (7), et
un évidement (28) tubulaire axial est prévu pour former chaque fois une masse polaire (7) à forme tubulaire, et de l'air de refroidissement étant susceptible d'être introduit à travers l'évidement (28).

3. Agencement de bobine magnétique selon la revendication 1 ou la revendication 2, **caractérisé en ce que** les faces d'extrémité de masse polaire (29) sont chacune couvertes par une plaque de masse polaire (30), et la plaque de masse polaire (30), en formant une zone d'une paroi de fond de caisson de bobine (38), située du côté de l'interstice de champ, dépasse en pourtour latéralement la face d'extrémité de masse polaire (29) chaque fois associée, et
des séparateurs (33) sont introduits entre une face d'extrémité de masse polaire (29) et la plaque de masse polaire (30), pour former des entrefers isolation/refroidissement (32).

4. Agencement de bobine magnétique selon la revendication 3, **caractérisé en ce que** la plaque de masse polaire (30), dans la zone de la face d'extrémité de masse polaire (29), présente une cavité (31) à épaisseur de matériau plus faible que dans la zone de la paroi de fond de caisson de bobine (38), cavité dans laquelle pénètre la face d'extrémité de masse polaire (29), sachant que l'arête de pourtour de la face d'extrémité de masse polaire (29) est arrondie, et que les entrefers isolation/ refroidissement (32) s'achèvent à cet endroit en un intervalle annulaire (34), faisant office de sortie d'air de refroidissement.

5. Agencement de bobine magnétique selon l'une des revendications 1 à 3, **caractérisé en ce que**
les masses polaires (7, 8) ont, observées en vue de dessus, une forme circulaire ronde et sont placées à l'opposé l'une de l'autre, espacées à la distance de l'entrefer d'exposition à un champ (12), avec des faces circulaires de masse polaire, tournées les unes vers les autres, orientées parallèlement, faisant office de faces d'extrémité de masse polaire,
**en ce que** les bobines magnétiques (22) sont réalisées de manière correspondant à une forme en anneau circulaire,
**en ce qu'**un caisson de bobine (9, 10) entoure, en forme de parallélépipède, l'extrémité de masse polaire associée et la bobine magnétique (2) entourant cette extrémité de masse polaire, et
**en ce que** la au moins une ouverture d'entrée d'écoulement d'air de refroidissement (51, 52, 53) est disposée dans une paroi latérale de caisson de bobine (43) et/ou en au moins une zone d'angle d'une paroi de couvercle de caisson de bobine (47).

6. Agencement de bobine magnétique selon l'une des revendications 1 à la revendication 5, **caractérisé en ce que**
la bobine magnétique (22) présente une structure d'appui (40) pour les enroulements de bobine (39), de manière que,
dans la zone de la bobine magnétique (22), sont prévus des supports de bobine (41) côté fond, en forme de nervure, et, disposés au-dessus, des supports de bobine (42) côté couvercle en forme de nervure, réalisés sous forme de paires de supports de bobine (41, 42) associées, qui sont disposées décalée angulairement l'un par rapport l'autre, et radialement en forme de rayons,
**en ce que** des supports de bobine (41, 42), associées par paires, sont reliées respectivement par des bases isolantes (59), espacées radialement et orientées à peu près axialement, et
**en ce que**, de ce fait, entre les barres isolantes (59), sont formés des compartiments de logement (60), dans lesquels les enroulements de bobine (39), s'étendant en forme d'hélicoïde, sont logés et maintenus, de manière que,
du fait de la hauteur de nervure des supports de bobine (41) situés côté fond, les enroulements de bobine (39) sont soulevés par rapport à la paroi de fond (38) du caisson de bobine, en formant un intervalle annulaire d'entrée d'air de refroidissement (49) radialement extérieur, l'air de refroidissement étant susceptible d'être guidé encore par des intervalles intermédiaires déterminés par les barres isolantes (59), et les zones (66), entre les supports de bobine (42) côté couvercle, formant des ouvertures de sortie d'air de refroidissement, au-dessus des enroulements de bobine (39) non couverts.

7. Agencement de bobine magnétique selon la revendication 6, **caractérisé en ce que** les barres isolantes (59) sont réalisées sous forme de barres rondes en céramique.

8. Agencement de bobine magnétique selon la revendication 6 ou la revendication 7, **caractérisé en ce que** la paroi de fond de caisson de bobine (38), les parois latérales de caisson de bobine (43, 44, 45, 46), la paroi de couvercle de caisson de bobine (47), ainsi qu'une paroi intérieure d'une boîte à vent (48) à paroi mince, entourant radialement extérieurement la bobine magnétique (22), paroi espacée de la paroi de fond de caisson de bobine (38) de la valeur de l'intervalle annulaire d'entrée d'air de refroidissement (49), forment une boîte à vent (50) de pourtour, et
**en ce que** la bobine magnétique (22) est entourée radialement intérieurement par une paroi de guidage d'air (55), faisant qu'un intervalle annulaire (24, 57), pour la sortie de l'air de refroidissement de masse polaire, est créé entre cette paroi de guidage d'air (55) et une paroi de masse polaire (56) voisine.

9. Agencement de bobine magnétique selon l'une des revendications 6 à 8, **caractérisé en ce que** les supports de bobine (41, 42) sont d'allure cunéiforme, de sorte que les enroulements de bobine (39) radialement extérieurs, par leurs zones d'enroulements de bobine situées côté fond, sont soulevés plus encore de la paroi de fond de caisson de bobine (38) que les zones d'enroulement de bobine situées radialement plus intérieurement.

10. Agencement de bobine magnétique selon la revendication 9, **caractérisé en ce que**, en partant de l'intervalle annulaire d'entrée d'air de refroidissement (49), dans la zone cunéiforme, entre les supports de bobine (41) situés côté fond, s'étendent des plaques de guidage d'air (61, 62) à peu près parallèles au fond, sachant que, une plaque de guidage d'air (62), plus proche du fond, est respectivement plus longue et plus large qu'une plaque de guidage d'air (61) associée, située respectivement au-dessus.

11. Agencement de bobine magnétique selon la revendication 10, **caractérisé en ce que** les plaques de guidage d'air (61, 62) sont reliées à la paroi de fond de caisson de bobine (38) par des colonnes d'espacement (63) et/ou des bagues d'espacement.

12. Agencement de bobine magnétique selon l'une des revendications 6 à 11, **caractérisé en ce que** les supports de bobine présentent des alésages de logement (65), dans lesquels les barres isolantes (59) sont enfichées et maintenues,
**en ce que** les supports de bobine (41), situés côté fond, sont vissés et/ou collés à la paroi de fond de caisson de bobine (38),
**en ce que** les supports de bobine (42), situés côté couvercle, sont vissés radialement extérieurement de façon désolidarisable à la paroi de couvercle (47) et sont vissés radialement intérieurement de façon désolidarisable à des colonnes d'appui (64) montées sur la paroi de fond de caisson de bobine (38),
**en ce que**, au moyen de liaisons vissées et/ou collées, entre la paroi de fond de caisson de bobine (38), les parois latérales de caisson de bobine (43, 44, 45, 46) et la paroi de couvercle de caisson de bobine (47), existe une structure à caisson de bobine stable, susceptible d'être reliée à une partie de culasse magnétique voisine, par des éléments de liaison.

13. Agencement de bobine magnétique selon l'une des revendications 1 à 12, **caractérisé en ce que** la culasse magnétique (2) est réalisée en forme de M entant qu'agencement à trois branches, avec deux parties de culasse verticales (3, 4) parallèles espacées, de même géométrie, et avec deux parties de culasse transversales (5, 6), raccordées entre elles, avec chaque fois des masses polaires (7, 8), disposées en leur centre et orientées les unes vers les autres, parmi lesquelles au moins une partie de culasse transversale (5, 6) est manoeuvrable avec la masse polaire (7,8) raccordée et la bobine magnétique (22) associée, sous la forme d'un ensemble de construction, par rapport à une autre partie de culasse transversale (5, 6), pour le réglage de la valeur de l'entrefer d'exposition à un champ (12).
